# EUROPEAN PATENT APPLICATION

(11) **EP 3 779 421 A1**
(43) Date of publication of application: **17.02.2021**
(21) Application number: 19774495.6
(22) Date of filing: 25.03.2019
(51) Int. Cl.: G01N 27/02, G01N 33/483

(54) **BIO-SENSOR**

(30) Priority: 30.03.2018 JP 2018066633
(71) Applicant: Provigate Inc., Tokyo 113-0033 (JP)
(72) Inventor: YANAGIMOTO, Yoshiyuki, Tokyo 113-0033 (JP); KAJISA, Taira, Tokyo 113-0033 (JP)
(74) Representative: Michalski Hüttermann & Partner Patentanwälte mbB
(86) International application number: PCT/JP2019/012359
(87) International publication number: WO 2019/188902

(57) **Abstract**

Provided is a bio-sensor for detecting a substance to be measured that is contained in a solution, wherein the bio-sensor is provided with a sensing base plate which detects the substance to be measured, and a molecular chain which is disposed in a non-close-packed matter on the surface of the sensing base plate to inhibit substances not to be measured.

## Description

### TECHNICAL FIELD

The present disclosure relates to a biosensor.

### BACKGROUND ART

In recent years, various biosensors have been researched and developed, and are used in the fields of medicine, drug discovery, clinical examination, and the like. Biosensors utilize the excellent molecular identification ability that organisms have to recognize information (e.g., chemical elements) from the outside world as physical signal, and there are various principles and measurement objects. More specifically, a biosensor is a kind of chemical sensor that measures a chemical substance as a target, and is composed of a molecular identification element that recognizes the measurement target substance, and a signal conversion element that converts the information that a recognition has been done into a physical signal such as an electrical signal. Generally, since biomolecules such as enzymes, antibodies, DNA, cells, microorganisms, and compounds that capture biomolecules or the like are used for the molecular identification element, these sensors are called biosensors.

Various substances are contained in a sample other than chemical substances as markers, including a liquid derived from a living body such as a body fluid, and this becomes a measurement noise or a false signal, which affects measurement accuracy. In addition to the measurement target derived from a living body, it is known that substances present in a solution similarly reduce measurement accuracy or inhibit improvement in measurement accuracy.

### SUMMARY OF INVENTION

According to an embodiment of the present disclosure, there is provided a sensor for detecting a measurement target substance contained in a solution, the sensor comprising a sensing substrate for detecting the measurement target substance, and molecular chains arranged non-closely (not closest packed) on a surface of the sensing substrate and inhibiting the non-substance not to be measured.

A "solution" may be a body fluid. The solution may comprise a mediator.

The "body fluid" may be lymph fluid, tissue fluid such as interstitial fluid, intercellular fluid, interstitial fluid, and the like, and may be body cavity fluid, serosal fluid, pleural fluid, ascites fluid, pericardial effusion, cerebrospinal fluid, joint fluid (synovial fluid), and aqueous humor of the eye (aqueous). The body fluid may be digestive fluid such as saliva, gastric juice, bile, pancreatic juice, intestinal fluid, etc., and may be sweat, tears, nasal mucus, urine, semen, vaginal fluid, amniotic fluid, milk, etc. The solution may be a physiological buffer such as phosphate buffered saline (PBS) or N-tris(hydroxymethyl)methyl-2-aminoethanesulfonic acid buffer (TES), containing a measurement target substance. The solution is not particularly limited as long as the measurement target substance is contained.

"Measurement target substance " means a substance such as a molecule or an ion or a measurement target substance that is to be measured. "Non-measurement target substance " means a substance other than the measurement target substance and other than the substance that is an object of measurement. The "solution" may include a measurement target substance. When the measurement target substance is not measured, for example, during calibration or cleaning, the measurement target substance may not be included. The measurement target substance may be an inorganic molecule, an organic molecule, a biomolecule, or a bound molecule thereof. The measurement target substance may be one kind or a plurality of kinds. For example, the solution may be tears and the measurement target substance may be glycoalbumin (glycated albumin) contained in the tears. Alternatively, the measurement object may be glucose in blood or serum, albumin, glycoalbumin, uric acid, glycated hemoglobin, glucose in interstitial fluid, glucose in tears, albumin, urinary albumin, glucose, etc. The measurement target substance may be a nucleic acid.

The "sensor" may be a biosensor or a biomolecular sensor. The sensor may be a protein sensor or an albumin sensor. The sensor may be an electrochemical sensor. The electrochemical sensor may be a potentiometric sensor, an amperometric sensor, an impedance sensor. The sensor may be a plasmon sensor or an optical sensor. The sensor may detect the presence of a measurement target substance, may measure, estimate, and determine a parameter, such as the concentration of the measurement target substance, and may measure a time change in the parameter, such as the concentration of the measurement target substance.

At the "electrode", the electrical state changes due to approach, contact, adsorption, bonding, etc. of the measurement substance. Alternatively, the electrodes are configured such that a change in the electrical state or an electrical change occurs due to the measurement target substance or the like approaching.

In some embodiments, the change in the electrical state of the electrode may be a change in charge or potential due to the transfer of electrons generated when the measurement target substance binds to the electrode surface. For example, a charge generated when a biomolecule such as proteins having a cysteine site, a thiol group or an SH group is bound to an atom of an electrode (e.g., a gold atom) may be detected. For example, a protein having a thiol (SH) group, which is a measurement target substance, adsorbs on the surface of gold, which is an electrode, and Au-S bonding forms.

In another embodiment, an opposite charge that occurs when a charged biomolecule approaches the vicinity of electrode may be detected.

In yet another embodiment, the solution includes a mediator other than the measurement target substance, and the charge when the mediator undergoes a redox reaction at the electrode may be detected. The temporal measurement or the temporal change of the charge may be detected as a current. For example, the effective area of the electrode is reduced by adsorption, bonding, or the like of the measurement target substance to the electrode. In the electrode, a current flows in a manner corresponding to the amount of the measurement target substance that has come close to the electrode. The mediator may be a metal complex or a transition metal complex. The mediator may be a divalent or trivalent iron (Fe) ion (Fe²⁺, Fe³⁺), chromium (Cr) ion (such as Cr³⁺, Cr⁴⁺), cobalt (Co) ion (such as Cr²⁺, Cr³⁺), nickel (such as Ni2⁺, Ni3⁺) ion, palladium (Pd) ion (such as Pd²⁺, Pd⁴⁺).

In some embodiments, the electrode may comprise a material having electrical conductivity. For example, the electrode may be formed of a metal, a noble metal such as gold, silver, platinum, copper, palladium, or an alloy of metals. The electrodes may comprise a conductive metallic oxide, such as ITO, IGZO, etc., and may comprise carbon materials, such as graphene.

The "sensing substrate" may be a sensing portion or a substrate. The sensing substrate may be an electrode or a sensing electrode. The presence of the measurement target substance or the influence of the non-measurement target substance may be detected electrically or by other methods.

In another embodiment, the sensing substrate may not be an electrode that is electrically conductive. The measurement may be performed by an electrical method using a material which is not generally called an electrode. The sensing substrate may be composed of or include a semiconductor or a non-conductive material. For example, the sensing substrate may comprise an insulating metal oxide (such as a Ta₂O₅, SiO₂). The insulating sensing substrate can be used for impedance measurement, for example.

Sensing may be performed using plasmons or light, or a combination of a plurality of them, in addition to electrical sensing. For example, the sensing substrate may be irradiated with light from, for example, the back surface, to detect a change in the refractive index. The refractive index changes depending on the measurement target substance adsorbed on the sensing substrate or the like.

The "molecular chain" may be an inorganic molecular chain and may be an organic molecular chain. As an example, the inorganic molecular chain may be a molecular chain formed by crosslinking of polydimethylsiloxoic acid.

The molecular chain may at least partially have hydrophilicity, and at least one end toward the solution or its vicinity may have hydrophilicity. When a molecular chain has hydrophilicity, particularly when an end portion of a molecular chain or a vicinity thereof has hydrophilicity, a protein having a charge is elasticated by a site having hydrophilicity, so that it becomes difficult to approach a sensing substrate or an electrode.

The molecular chain may be a hydrophilic polymer. The hydrophilic monomer may be polymerized to prepare a hydrophilic polymer. The molecular chain may be a molecular chain in which a polymerization initiating molecule and a hydrophilic polymer are bonded. Hydrophilic polymers may, for example, include cellulose derivatives such as 2-methacryloylxyethyl phosphorylcholine (MPC), hydroxyethylmethacrylate, ethylene glycol methacrylate, hydroxypropylmethylcellulose (HPMC), sodium carboxymethylcellulose (CMC-Na), hydroxyethylcellulose (HEC); polysaccharides such as alginic acid, hyaluronic acid, agarose, starch, dextran, and pullulan and their derivatives; homopolymers such as carboxyvinyl polymers, polyethylene oxide, poly(meta)acrylamide, copolymers of such homopolymers with polysaccharides, etc., and copolymers of such homopolymers with other monomers; proteins such as collagen, gelatin, etc. and their derivatives; heparin, hyaluronic acid, chondroitin sulfate, dermatan sulfate, dextran sulfate, keratan sulfate, glycosaminoglycans such as heparan sulfate, polysaccharides such as chitin and chitosan, and mucopolysaccharides.

The organic molecular chain may be formed using a technique such as a radical polymerization method or a living radical polymerization method. In some embodiments, organic molecular chains may be formed using an atom transfer radical polymerization method (Atom Transfer Radical Polymerization, ATRP). In some embodiments, an organohalogen compound may first be formed as a polymerization initiator on a metal electrode surface, and a hydrophilic molecular chain of a desired length may be formed on this by ATRP.

This organohalogen compound may be a molecule having a thiol group or a disulfide group which forms a relatively strong bond with a metal such as gold, silver, platinum, or copper at one end, , and having a halogen atom such as bromine (Br) at the other end, or a thiol derivative or a disulfide derivative. For example, thiol groups form S-Au bonds at the gold surface, and molecular chains can stand on the gold surface. The length of the molecular chain of an organohalogen compound is defined by the number of carbon atoms. The carbon number may be adjusted. For example, the number of carbon atoms may be 6, 11, 10, 11, 16, or the like. If the number of carbon atoms is too short, there is a possibility that when forming on the electrode surface, the molecular chains will not interact with each other, making it difficult to form at an appropriate density. If the number of carbons is too long, the molecular chain may become too dense. When the electrodes are made of a conductive metal oxide (ITO, IGZO, or the like), a carbon material such as graphene, or an insulating metal oxide (Ta2O5, SiO2 or the like), a silane coupling agent having a halogen atom at one end may be used.

In the present disclosure, a molecular chain does not completely or closely cover an electrode surface in contact with a solution but rather covers a portion of an electrode surface in contact with a solution. Molecular chains may be partially disposed on the electrode surface. Alternatively, the molecular chains are arranged at a density less than the maximum density (close-packed) that can be arranged on the electrode surface in contact with the solution. Still another way, the molecular chains are arranged on the electrode surface so as to cover the electrode surface at a less dense or non-closest density at the electrode surface in contact with the solution. It is preferable that the density of the molecular chains on the electrode surface is a density which substantially allows the measurement target substance to reach the electrode surface and substantially inhibits the non-measurement target substance from reaching the electrode surface. The frequency of arrival of the measurement target to the electrode surface when the molecular chains are disposed may be reduced, as compared with a case where the molecular chains are not disposed on the electrode surface. Molecular chains may not be able to inhibit all types of non-measurement target substances. For example, molecular chains can substantially inhibit one or more types of non-measurement target substances.

The space between the molecular chains can allow substances of size equal to or less than them or desired measurement target substances to approach the electrode surface and block or inhibit substances of size equal to or greater or non-measurement target substances from approaching the electrode surface.

The density of the molecular chains can be controlled by the concentration of the polymerization initiating molecule, the type of solvent, the surface state of the electrode and the production conditions (for example, the conditions of sputtering (sputter deposition) for producing a gold electrode), the conditions of impregnation of the electrode into the solution, for example, the impregnation time, the temperature, and the like.

For example, the density of molecular chains of Au-S bond can be measured using cyclic voltammetry, which applies a voltage to an electrode and measure the charge released from Au-S as the molecular chain peels off or a current that is the temporal change of the charge. Alternatively, the density of the molecular chains can be determined by atomic force microscopy (AFM), scanning tunneling microscopy (STM), etc. Methods for measuring the density of molecular chains are not limited to these, and other methods may be used.

Besides the density, the filtering characteristics and selectivity of the measurement target substance and the non-measurement target substance can be adjusted by properties such as length, type, hydrophilicity, and charge of the molecular chain.

"Inhibition" may completely inhibit the reaching of the non-measurement target substance to the electrode surface, or may inhibit a portion thereof. "Inhibition" means suppressing approach, contact, binding, or the like the non-measurement target substance to the electrode, or reducing the probability of reaching the electrode surface. Alternatively, "inhibition" may mean avoiding or reducing the influence of the non-measurement target substance to such an extent that the detection and quantification of the measurement target substance can be sufficiently performed, or to such an extent that the target substance can be sufficiently selectively detected, or the like.

All of the measurement target substances may reach the electrode without being blocked by the molecular chains, or a portion thereof may reach the electrode. For example, when measuring albumin in tears, it is believed that many of the proteins in tears containing albumin are affected by molecular chains. There may be albumin molecular chains that are repelled by the molecular chains and cannot reach the metal surface.

According to some embodiments, the sensor may further comprise an electrical circuit (a measuring circuit, a measuring instrument/device, a detector) connected to the electrode or the sensing electrode and detecting a change in electrical condition at the electrode by the measurement target substance. In another embodiment, the sensor may be configured to be electrically connected to an electrical circuit. The sensors may be configured to be removable from each other with respect to another unit that includes electrical circuitry. The sensor may be a sensor chip.

The electrical circuit may be a potential measuring circuit. The potential measuring circuit may include a potentiostat. The potential measuring circuit may include a field effect transistor (FET). The electrode is connected to the gate of the FET, and the potential at the electrode can control the current between the source and the drain of the transistor. In another embodiment, the electrical circuit may be a current measuring circuit, an impedance measuring device, or an amperometric measuring device.

For example, when an impedance measurement is performed using a mediator contained in a solution, the mediator passes charge to the working electrode by a redox reaction upon contact with the electrode. This is detected or measured as a current by a current measuring circuit. When the measurement target substance reaches and is adsorbed or binds on the working electrode, the substantial area of the electrode decreases, that is, the surface area of the electrode to which the mediator can reach decreases. Therefore, generally, the amount of the non-measurement target substance reaching the electrode increases with time, the real area of the electrode decreases, and the current value (I) by the mediator drops. The concentration of the measurement target substance in the solutions can be obtained from the slope (dl/dt) of the drop of the current value, the drop (ΔI) of the current value (I) up to a certain predetermined time, or the like. Alternatively, the measurement target substance in the vicinity of the electrode surface changes the electric double layer or the like to cause a change in the capacitance component of the interface. That is, the impedance of the equivalent circuit at the interface changes. Therefore, by measuring the phase difference of the alternating current, the amount of the measurement target substance in the vicinity of the electrode or the concentration of the measurement target substance in the solution can be obtained by conversion, estimated, and specified.

According to the present disclosure, potentially or by way of example, detection or measurement of measurement target substances at relatively low concentrations becomes possible even in the presence of non-measurement target substances.

### BRIEF DESCRIPTION OF DRAWINGS

FIG. 1 is a schematic diagram illustrating a configuration of a sensor according to a first embodiment of the present disclosure.
FIG. 2 is a schematic diagram illustrating a configuration of a sensor according to a second embodiment of the present disclosure.
FIG. 3 is a schematic diagram illustrating a configuration of a sensor according to a third embodiment of the present disclosure.
FIG.4 is a schematic diagram illustrating a configuration of a sensor according to a fourth embodiment of the present disclosure.
FIG.5 is a schematic diagram illustrating a configuration of a sensor according to a fifth embodiment of the present disclosure.
FIG.6 is a schematic diagram illustrating a configuration of a sensor according to a sixth embodiment of the present disclosure.
FIG.7 is a conceptual diagram illustrating an example of the formation of molecular chains.
FIG.8 is a graph showing the measurement results of the proteins in the pseudo tear fluid.
FIG.9 is a graph showing the measurement results of the proteins in the pseudo tear fluid.
FIG. 10 is a graph showing the correlation between the concentration of albumin and the amount of surface potential change.

### DETAILED DESCRIPTION OF THE INVENTION

### <First Embodiment>

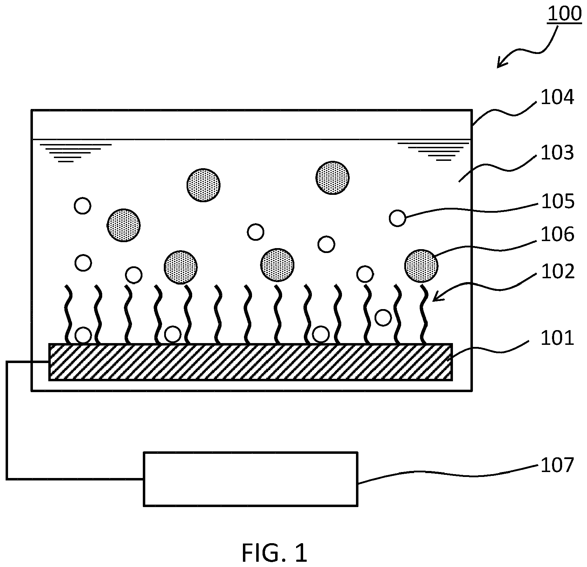

FIG. 1 is a schematic diagram illustrating a configuration of a biosensor 100 according to an embodiment (first embodiment) of the present disclosure. The biosensor 100 shown in FIG. 1 has an electrode 101 and molecular chains 102 non-closely disposed on the electrode 101. The biosensor 100 has a container 104 containing a solution 103. The electrode 101 and the molecular chains 102 are disposed in the container 104. The solution 103 includes measurement target substances 105 and non-measurement target substances 106. Due to the presence of the molecular chains 102, it becomes difficult for the non-measurement target substances 106 to reach or approach the surface of the electrode 101. The measurement target substances 105 can substantially reach the surface of the electrode 101.

Due to the presence of the molecular chains 102, a smaller amount of the measurement target substances 105 than the amount that would have reached the surface of the electrode 101 without the molecular chain 102 may reach the surface of the electrode 101. In other words, there may be measurement target substances 105 which cannot reach on the surface of the electrode 101 due to the influence of the molecular chains 102. A part of the measurement target substances 105 may reach the surface of the electrode 101. The non-measurement target substances 106 are substantially inhibited from reaching the surface of the electrode 101 by the molecular chains 102. A part of the non-measurement target substances 106 may reach the surface of the electrode 101.

The sensor 100 shown in FIG. 1 further comprises an electrical circuit or measurement circuit 107 electrically connected to the electrode 101 to detect the electrical condition of the electrode 101. The electrical state of the electrode 101 changes due to the approach, contact, and the like of the measurement target substances 105. The electrical circuit 107 can detect the electrical change.

### <Second Embodiment>

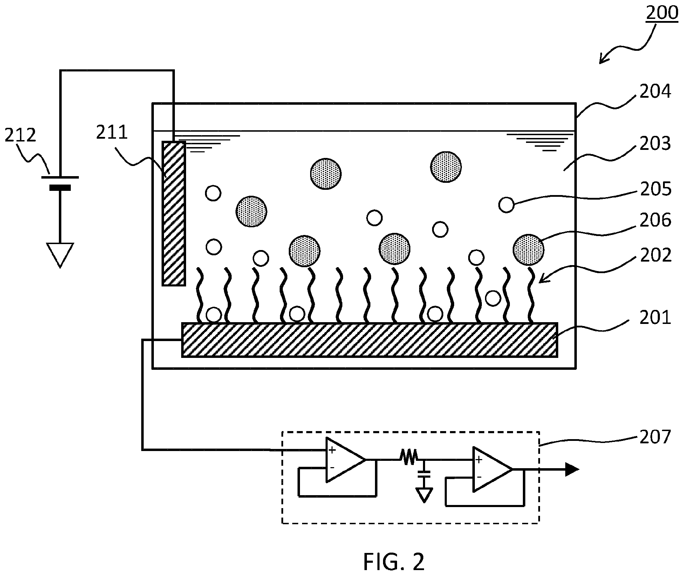

FIG.2 is a schematic diagram illustrating a configuration of a biosensor 200 according to one embodiment of the present disclosure (second embodiment). The biosensor 200 shown in FIG. 2 has an electrode 201 and molecular chains 202 disposed on the electrode 201. The biosensor 200 has a container 204 containing a solution 203. The electrode 201 and the molecular chains 202 are disposed in the container 204. The solution 203 includes measurement target substances 205 and non-measurement target substances 206. The sensor 200 shown in FIG. 2 further comprises a surface potential measuring device 207 connected to the electrode and a reference electrode 211. The surface potential measuring device 207 shown in FIG. 2 is configured to have a first stage of the operational amplifier, a low-pass filter and a second stage of the operational amplifier. The surface potential measuring device 207 may further include an A/D converter, or may be connected to an A/D converter. The sensor 200 shown in FIG. 2 further includes a reference electrode 211. The reference electrode 211 is disposed in the container 203 and is configured to contact solution 203. The reference electrode 211 shown in FIG. 2 is connected to a power supply 212 that provides a reference voltage.

### <Third Embodiment>

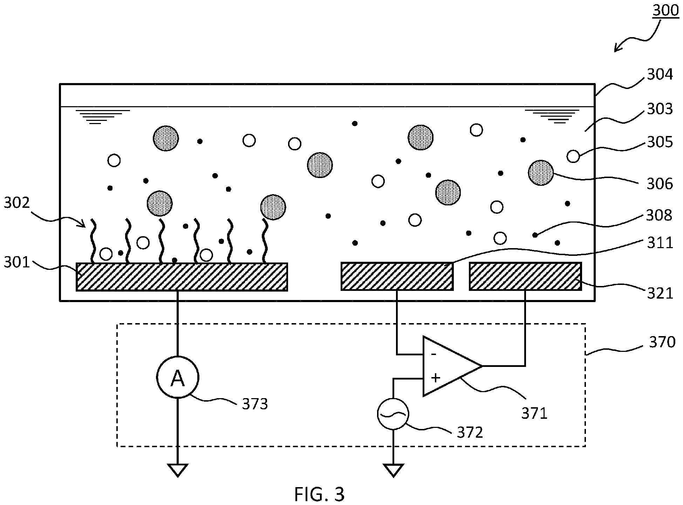

FIG.3 is a schematic diagram illustrating a configuration of a biosensor 300 according to one embodiment of the present disclosure (third embodiment). The biosensor 300 includes a working electrode 301, a reference electrode 311, and a counter electrode 321 in a container 304. Molecular chains 302 are disposed on a surface of the working electrode 301 in contact with the solution 303, and is configured so as to inhibit the non-measurement target substances 306 contained in the solution 303 from approaching the working electrode 301 and selectively allowing the measurement target substances 305 to approach the electrode. The working electrode 301 is connected to a current measuring circuit 373.

The reference electrode 311 and the counter electrode 321 shown in FIG. 3 are connected to an operational amplifier 371. More specifically, the reference electrode 311 is connected to the inverting input (-IN) of the operational amplifier 371, and the counter electrode 321 is connected to the output (OUT) of the operational amplifier 371. An AC power supply 372 is connected to the non-inverting input (+IN) of the operational amplifier 371.

The configuration of FIG. 3 may be referred to as a so-called three-electrode measurement system. When a voltage is applied from the power supply 372, a feedback loop is formed which returns from the output of the operational amplifier 371, via the counter electrode 321, the solution 303, and the reference electrode 311, and back to the inverting input (- IN) of the operational amplifier 371. By the action of this feedback loop, the voltage applied to the counter electrode 321 is controlled such that the voltage transmitted from the reference electrode 321 to the counter electrode 321 is equal to the reference voltage provided by the power supply 372. Although the operational amplifier 371 is one component of the feedback circuit, other circuit components having similar functions may be used in another embodiment.

The solution 303 shown in FIG. 3 includes mediators 308 such as iron ions (Fe2+ or Fe3+). When the mediator 308 contacts the working electrode 301, the charge is passed to the working electrode 301 by a redox reaction. This is detected or measured as a current by the current measuring circuit 373. When the measurement target substances 305 reach and are adsorbed or bound on the working electrode 301, a substantial area of the working electrode 301 decreases, that is, the surface area of the working electrode 301 that can be reached by the mediators 308 decreases. Therefore, in general, the amount of the non-measurement target substances 306 that have reached the working electrode 301 increases with time, the substantial area of the working electrode 301 decreases, and the current value (I) by the mediator 308 drops. The concentration of the measurement target substance 305 in the solution 303 can be estimated or specified from the slope (dl/dt) of the drop of the current value, the drop amount (ΔI) of the current value (I) up to a certain predetermined time, or the like. Alternatively, the measurement target substances 305 in the vicinity of the surface of the electrode 301 change the electric double layer or the like to cause a change in the capacitance component of the interface. That is, the impedance of the equivalent circuit at the interface changes. Therefore, by measuring the phase difference between the current value and the alternating current, the amount of the measurement target substances 305 in the vicinity of the electrode 301 or the concentration of the measurement target substances 305 in the solution 303 can be obtained by conversion. The above description of the measurement mechanism is illustrative and other mechanisms and measurement principles may be used.

In the system shown in Fig. 3, while the impedance measurement by applying an AC voltage is performed, in other embodiments, a DC power supply may be used instead of the AC power supply 372, to perform amperometry measurements.

With respect to the electrode 301 on which the molecular chains 302 are disposed, a similar electrode may be further provided as a control electrode without molecular chains arranged. By comparing the signal from the electrode via the molecular chain with the signal from the electrode without the molecular chain (current value, potential value, etc.) or taking the difference between them, it is possible to remove or reduce the noise due to the influence of low molecular substances, ions, etc. which cannot be inhibited by the non-measurement target substance or the molecular chain in the solution. For example, when albumin contained in tears is measured as a target, small molecules such as ascorbic acid or uric acid can be sensed in tears by an electrode on which molecular chains are arranged. Signals from these non-measurement target substances can cause noise. Using the difference from the signal from the electrode without the molecular chains these noises can be reduced and the sensitivity and efficiency of the intended measurement can be improved.

### <Fourth Embodiment>

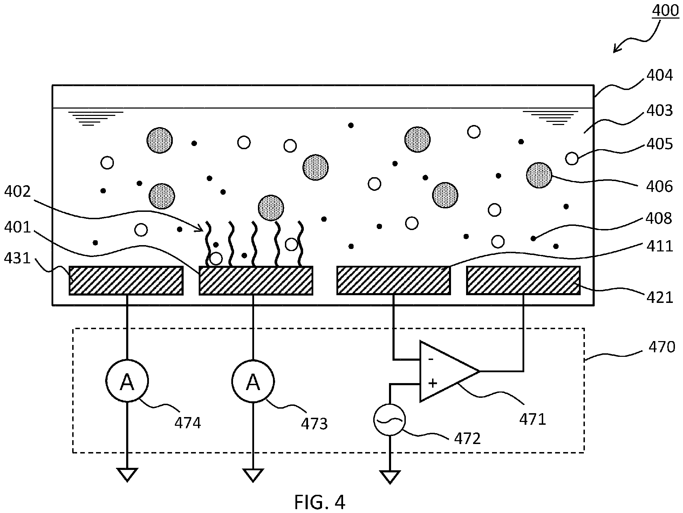

FIG.4 is a schematic diagram illustrating a configuration of a biosensor 400 according to one embodiment of the present disclosure (fourth embodiment). The biosensor 400 includes a sensing working electrode 401, a control working electrode 431, a reference electrode 411, and a counter electrode 421, in a container 404. Molecular chains 402 are disposed on a surface of the sensing working electrode 401 in contact with the solution 403 so as to inhibit the non-measurement target substances 406 from approaching the working electrode 401. The sensing working electrode 401 and the control working electrode 431 are connected to current measuring circuits 473 and 474, respectively. Although this is a configuration of the three electrode method as in the third embodiment shown in FIG. 3, the sensor 400 according to the present embodiment includes a working electrode that does not have a molecular chain, or a working electrode 431, in addition to the sensing working electrode 401 including the molecular chains 402. The difference of the measurements from the current measurement circuits 473, 474 can be taken. The difference may be performed by analog or digital.

The reference electrode 411 and the counter electrode 421 shown in FIG. 4 are connected to an operational amplifier 471. More specifically, the reference electrode 411 is connected to the inverting input (-IN) of the operational amplifier 471 and the counter electrode 421 is connected to the output (OUT) of the operational amplifier 471. A voltage power supply 472 is connected to the non-inverting input (+IN) of the operational amplifier 471. The configuration and function of the feedback circuit is similar to the third embodiment.

While in FIG. 4 impedance measurements are performed by applying an AC voltage, in other embodiment, a DC power supply can be arranged instead of the AC to perform amperometry measurements (not shown).

### <Fifth Embodiment>

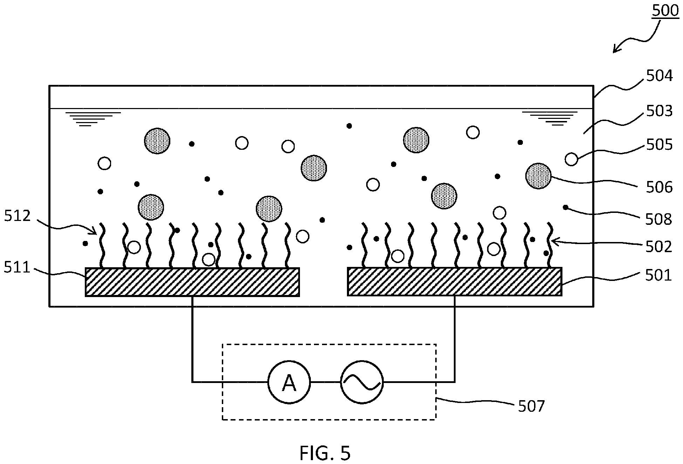

FIG.5 is a schematic diagram illustrating a configuration of a biosensor 500 according to one embodiment of the present disclosure (fifth embodiment). Solution 503 includes measurement target substances 504, non-measurement target substances 505, and mediators 508. The biosensor 500 shown in FIG. 5 includes two electrodes (sensing electrodes) 501 and 511 that are paired with each other. On the first electrode 501 and the second electrode 511 disposed in the container 504, the first molecular chains 502 and the second molecular chains 512 are disposed respectively. The first electrode 501 and the second electrode 511 are connected to an impedance measuring device (measurement circuit) 507. For example, the concentration of the measurement target substances 505 in the solution 503 can be obtained from the phase difference between the current value and the current when an AC electric field is applied. The first molecular chains and the second molecular chains are configured to inhibit the non-measurement target substances 506 from binding to the electrodes 501, 511, respectively. In some embodiments, a plurality of pairs of the first electrodes 501 and the second electrodes 502 may be provided.

The solution 503 shown in FIG. 5 includes mediators 508 such as iron ions (Fe2+ or Fe3+). When the mediator 508 contacts the electrodes 501,511, the charge is passed to the electrodes 501,511 by a redox reaction. This is detected or measured as a current by the current measuring circuit 507. When the measurement target substances 505 reach and are adsorbed or bound to the electrodes 501 and 511, a substantial area of the electrodes 501 and 511 is decreased, and an electric double layer or the like is changed to cause a change in the capacitance component of the interface. That is, the impedance of the equivalent circuit at the interface changes. Therefore, by measuring the phase difference between the current value and the alternating current, the amount of the measurement target substances 505 in the vicinity of the electrode 501 or the concentration of the measurement target substances 505 in the solution 503 can be obtained by conversion. The above description of the measurement mechanism is illustrative and other mechanisms and measurement principles may be used.

The two-electrode system operating in conjunction with each other as in FIG. 5 can reduce the effects of drift of measurement parameters during a measurement, variations from measurement to measurement, how the electrodes are arranged, and so on. Furthermore, it can be manufactured relatively easy and compact.

### <Sixth Embodiment>

With respect to an electrode on which molecular chains are disposed, a similar electrode may be further provided as a control electrode without molecular chains arranged. By comparing the signal from the electrode via the molecular chains with the signal from the electrode without the molecular chains and taking the difference between them, it is possible to remove or reduce the noise due to the influence of the non-measurement target substance in the solution. For example, when albumin contained in tears is measured as a target, small molecules such as ascorbic acid or uric acid can be sensed in tears by an electrode on which are molecular chain are disposed. Signals from these non-measurement target substances can cause noise.

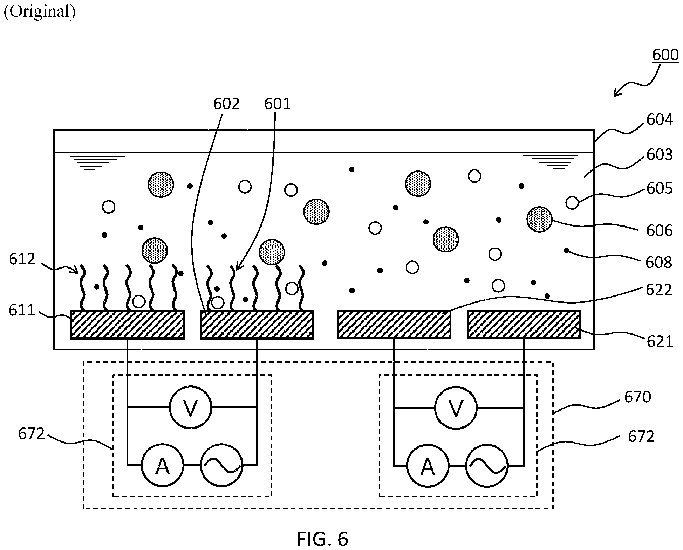

FIG.6 is a schematic diagram illustrating a configuration of a biosensor 600 according to one embodiment of the present disclosure (sixth embodiment). Solution 603 includes measurement target substances 604, non-measurement target substances 605, and mediators 608. Electrodes without molecular chains are added to the configuration of the fifth embodiment shown in FIG. 5. That is, in the biosensor 600 shown in FIG. 6, the sensing electrode pair disposed in the container 604, the first sensing electrode 601 and the second sensing electrode 611 have the first molecular chains 602 and the second molecular chains 612 that are arranged non-closely on the respective electrode surfaces. The first sensing electrode 601 and the second electrode 611 is connected to the impedance measuring device (measuring circuit) 671. The biosensor 600 of FIG. 6 further includes a control electrode pair, a first control electrode 621 and a second control electrode 631, which do not have molecular chains. The first control electrode 621 and the second control electrode 631 is connected to the impedance measuring device (measuring circuit) 672. The impedance measurement device 671 for sensing and the impedance measurement device 672 for control may be collectively referred to as an electrical measurement circuit 670.

In some embodiments, the impedance measuring device has an AC voltage source and a current measuring circuit, and the power measuring circuit may measure a current flowing between the first electrode and the second electrode. The AC power supply and the current measuring circuit may be arranged in series. In another embodiment, the impedance measuring device may have an alternating current source and a voltage measuring circuit. In FIG. 6, the impedance measuring device 671 for sensing and the impedance measuring device 672 for control are disposed as separate impedance measuring devices. However, in some embodiments, one impedance measuring device (not shown) may be provided and configured to be alternately or selectively connected to the control electrode pair and the sensing electrode pair via a switch. Thereby, the sensor or the electrical measurement system can be miniaturized.

### <Manufacturing Example>

Hereinafter, an example of a method of manufacturing a sensor having hydrophilic polymer molecular chains on a gold electrode surface will be described.

First, a gold thin film having a thickness of 100 nm (nanometers) was formed on a glass substrate in a region having a diameter of 10 mm, by a sputtering method. Then, polymerization initiating molecules were bonded on this gold electrode surface at one end thereof. Then, from the other end of this polymerization-initiated molecule, hydrophilic polymer molecular chains were formed by atom-transfer radical polymerization (ATRP method). Hereinafter, each step will be described in more detail.

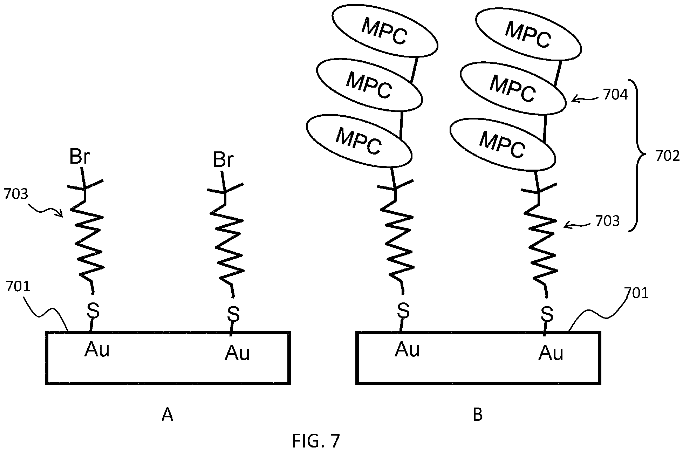

The formation of the polymerization initiating molecules on the gold electrode is as follows:
First, a glass substrate on which a gold electrode was formed was immersed in a 1 mM bis[2-(2-bromoisobutyryloxy)undecyl]disulfide/ethanol solution to bond polymerization initiating molecules on a gold electrode. Thus, as shown in FIG. 7A, the polymerization initiating molecule was immobilized by forming Au-S bonds with gold atoms of the electrode surface 701 at one end, thereby forming polymerization initiating molecular chains 703 having a length of about 10 carbon atoms having bromine (Br) at the free end which is the other end. This molecular chain is hydrophobic. The density of the molecular chains can be controlled by the immersion time in the above-mentioned bis[2-(2-bromoisobutyryloxy)undecyl]disulfide/ethanol solution (polymerization initiating molecular solution). In the following Examples 1 and 2, sensors in which the immersion time was 2 hours and 48 hours at room temperature were used. Thereafter, the substrate was removed from the polymerization-initiated molecular solution and washed with ethanol and pure water.

The formation of hydrophilic polymer molecular chains is as follows:
First, 16 mmol of 2-methacryloyloxyethyl phosphorylcholine (MPC) and 4 mmol of N-3-(dimethylamino)propylmethacrylamido were dissolved in 112 ml of dimethylformamide and 48ml of ultrapure water. Then 0.16 mmol of cupric bromide and 0.8 mmol of tris(2-pyridylmethyl)amine were added. Next, a solution was prepared in which 0. 8 mmol of ascorbic acid was added. In this solution (monomer solution), a glass substrate comprising a gold electrode to which a polymerization initiating molecule was bonded was immersed.

### **Chemical Equation 1**

Thereafter, the atmosphere around the monomer solution was maintained in a vacuum and subjected to a polymerization reaction at room temperature for 20 hours. This initiated the polymerization with bromine in Fig. 7A, forming molecular chains 702 having multiple MPCs (Fig. 7B). The portion of the MPC of the molecular chain 702 (hydrophilic portion 704) has hydrophilicity. The length of the MPC portion is determined by the temperature, time, MPC monomer concentration, and solvent of the polymerization reaction. In the above manufacturing method, a hydrophilic polymer 704 was formed in which about 1000 MPCs were linked.

Therefore, although the molecular chain 702 is hydrophobic at the portion 703 of the carbon chain on the fixed end side, it can be referred to as a hydrophilic polymer molecular chain. Since this hydrophilic portion is at the tip of the polymerization initiating molecular chain 703, less hydrophilic proteins are repelled, reducing the probability of approaching the electrode 701.

Subsequently, the gold thin film on which the hydrophilic polymer was formed was electrically connected to a potentiostat, which was a surface potential measuring device, via wiring.

Note that, in the sensor used in Comparative Example 1, the gold electrode was prepared in the same manner as in the sensor used in Example 1 and Example 2, but no molecular chain was formed. In other words, the sensor used in Comparative Example 1 is a sensor having a bare gold electrode, and it can be said that the sensor has a zero immersion time in a polymerization initiating molecular solution.

### <Example 1 and Comparative Example 1: Examples of Measuring the Exclusion Effect of Tear Proteins Other Than Albumin>

The surface of a gold electrode with a hydrophilic polymer made as described above was immersed in biological buffer (Phosphate-buffered saline, pH7 4) for 48 hours. Subsequently, as protein contained in tears, the following proteins were injected into a solution, and each concentrate was injected so that the concentration in the solution would become values as shown below. The injections were was performed in the following order.
Lysozyme 2.4 mg/mL
Lactoferrin 1.8 mg/mL
γ-globulin 0.5 mg/mL

These concentrations correspond to the concentrations in tears. The surface potential change accompanying the addition of these measurement target substances was measured by a real-time measuring apparatus.

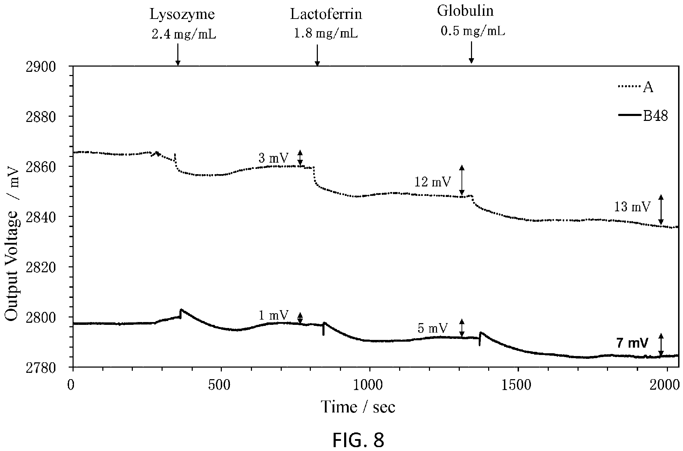

FIG. 8 shows the change in surface potential at the electrode over time with the addition of the tear proteins. The vertical axis of FIG. 8 shows the output power from the gold electrode (mV), the horizontal axis shows the measurement time (seconds). The dotted line (A) shows the measurement result of the case where there is no molecular chain on the gold electrode surface of the measurement result (Comparative Example 1), and the solid line (B48) shows the measurement result of the case where the molecular chain is provided on the gold electrode surface (Example 1). The display of both measurement results in FIG. 8 is adjusted so that the input time of each protein is uniform on the time axis and the readability is improved on the vertical axis. In Comparative Example 1 (dotted line A), the additions of lysozyme, lactoferrin, and γ-globulin resulted in potential changes of 3 mV, 12 mV, and 13 mV, respectively. In contrast, in Example 1 (solid line B48), the potential changes of 1 mV, 5 mV, and 7 mV were observed by additions of lysozyme, lactoferrin, and γ-globulin, respectively. That is, the potential changes of lysozyme, lactoferrin, and γ-globulin in Example 1 were about 1/2 to 1/3 of those in Comparative Example 1. In Example 1, it was found that due to the presence of molecular chains, these proteins were inhibited from arriving at the electrode surface. The hydrophilicity of the molecular chain is thought to function to repel the proteins.

### <Example 2: Detection and Quantification of Albumin in Pseudo-Tear>

In this example, hydrophilic polymer molecular chains on a gold electrode were formed at a plurality of densities to evaluate selectivity for a plurality of proteins. That is, the relationship between the density of the molecular chains and the potential change due to the arrival of each protein to the gold electrode was examined.

The concrete procedure leading to the measurement will be described again. First, as described in Manufacturing Example 1, a gold electrode was prepared on a glass substrate, and hydrophilic polymer molecular chains were made on the gold electrode. However, in this example, a plurality of densities of polymerization initiating molecules were prepared in accordance with the immersion time of a 1 mM bis[2-(2-bromoisobutyryloxy)undecyl]disulfide/ethanol solution. The longer the immersion time, the higher the density of the polymerization initiating molecules. In this Example, sensors of 2 kinds of hydrophilic polymer molecular chains (sensor B02 and sensor B48, respectively) having the immersion time of 2 hours and 48 hours and a gold electrode without the same molecular chain as in Comparative Example 1 (sensor A) were prepared.

Each electrode-surface was then filled with biological buffer (Phosphate-buffered saline, pH 7.4). Various proteins were subsequently added to this solution. The proteins used for the measurement and the concentration in solution after administration are as follows.

The addition to the solution was carried out in the order described below.
Lactoferrin 1.8 mg/mL
γ-globulin 0.5 mg/mL
   Human serum albumin 0.1 mg/mL
   Human serum albumin 1.1 mg/mL
   Human serum albumin 2.9 mg/mL
   Human serum albumin 6.8 mg/mL
   Human serum albumin 16.1 mg/mL

Then, the surface potential change caused by the addition of these proteins to the solution was measured by a real-time measuring device.

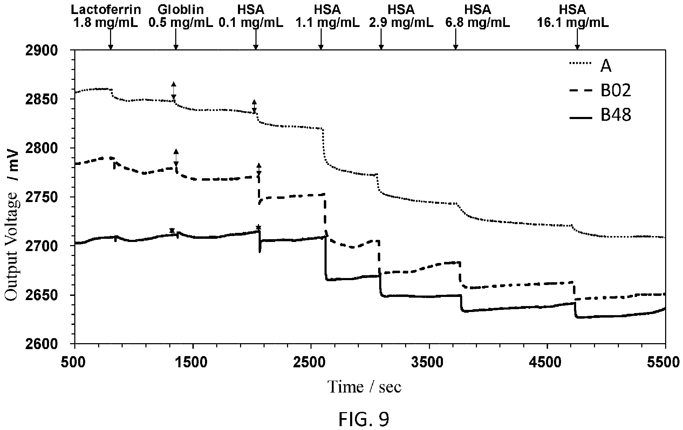

FIG. 9 shows the time evolution of the surface potential upon addition of each of the above proteins. The vertical axis of FIG. 9 shows the output voltage (mV), the horizontal axis shows the measurement time (seconds). The display of both measurement results in FIG. 8 is adjusted so that the input time of each protein is uniform on the time axis and the readability is improved on the vertical axis.

In the gold electrode without molecular chains (sensor A), the surface potential was detected from the addition of lactoferrin to the addition of the highest concentration of human serum albumin (HSA). It is considered that the change in the surface potential indicates a change in the surface potential due to the transfer of electrons when the Au-S bond is formed with the gold electrode. Surface potential change at the electrode were observed from the addition of lactoferrin to the addition of the highest concentration of human serum albumin (HSA) even with a sensor with a polymerization-initiated molecular immersion time of 2 h (sensor B02).

On the other hand, in the sensor (sensor B48) having a polymerization-initiated molecular immersion time of 48 hours, no substantial potential change at the electrode was observed with the addition of lactoferrin and globulin, and a surface potential change at the electrode was observed at the addition of HSA. It has been shown that a biosensor comprising hydrophilic polymer molecular chains as in this Example selectively detects the binding of albumin and gold as a surface potential change while reducing the influence of other proteins binding to the gold electrode. That is, it was confirmed that the sensor B48 does not detect lactoferrin and γ-globulin, but detects albumin. In other words, the sensor B48 may be said to have a selective detection sensitivity for albumin compared to lactoferrin, γ-globulin, and the like, among proteins in tear fluid. Such sensors can inhibit other proteins (non-measurement target substance) other than albumin, such as lactoferrin and γ-globulin, and can selectively detect albumin (measurement target substance). Such sensors can be used as albumin sensors in tears, as an example.

### <Quantification of Albumin>

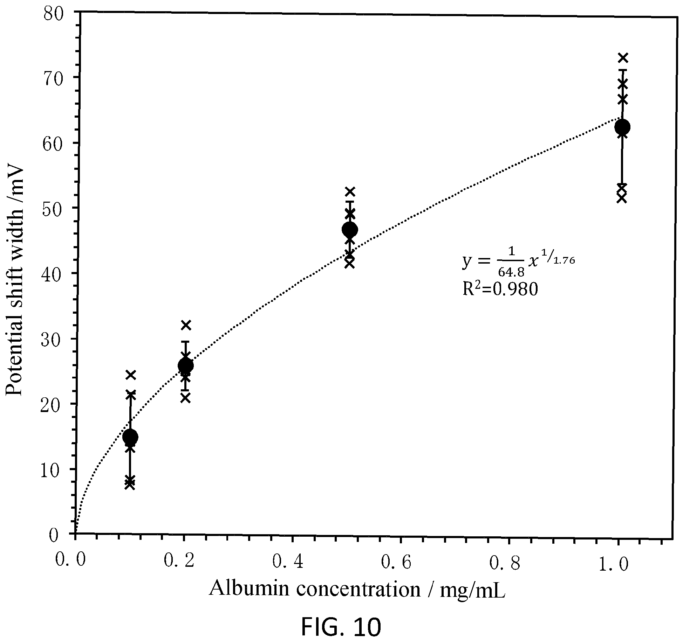

FIG. 10 is a diagram of plotting the collected concentrations of each albumin added as in FIG. 9, and the corresponding generated surface potential shift widths. As shown in FIG. 10, it was found that there is a substantially one-to-one correlation between the concentration of albumin and the amount of surface potential change. Therefore, it has been shown that the concentration of albumin can be quantified even in a pseudo tear solution containing proteins other than albumin using a sensor as shown in this example.

The present disclosure includes, but is not limited to, the following embodiments:
A1. A sensor for detecting a measurement target substance contained in a solution, comprising:
   a sensing electrode for electrically detecting the measurement target substance; and
   molecular chains arranged non-closely on the surface of the sensing electrode and inhibiting a non-measurement target substance.
A1b. The sensor of claim 1, wherein the molecular chain comprises a hydrophilic polymer.
A2. The sensor of claim A1 or A1b, further comprising an electrical measurement circuit connected to the sensing electrode for detecting an electrical state of the electrode.
A10. A sensor for detecting a measurement target substance contained in a solution, comprising:
   a sensing substrate; and
   molecular chains arranged non-closely on a surface of the sensing substrate and configured to inhibit a non-measurement target substance from approaching the surface of the sensing substrate.
A11. The sensor of claim A10, wherein the sensing substrate includes a sensing electrode that reflects a change in electrical state due to an approach of the measurement target substance.
A1c. A sensor for detecting a measurement target substance contained in a solution, comprising:
   a sensing electrode; and
   molecular chains arranged non-closely on a surface of the sensing electrode and configured to inhibit a non-measuring target substance from approaching the surface of the sensing electrode.
B1. A sensor for detecting a measurement target substance contained in a solution, comprising:
   a sensing electrode for electrically detecting the measurement target substance;
   molecular chains arranged non-closely on a surface of the sensing electrode and configured to inhibit a non-measurement target substance;
   a reference electrode; and
   a surface potential measuring device connected to the electrode.
C1. A sensor for detecting a measurement target substance contained in a solution, comprising:
   a pair of sensing electrodes configured to bond with the measurement target substance;
   molecular chains arranged non-closely on the sensing electrode and configured to inhibit a non-measurement target substance;
   an AC power supply for applying a voltage between the pair of sensing electrodes; and
   a sensing impedance measuring device connected between the pairs of sensing electrodes.
C2. The sensor of claim C1, further comprising:
   a pair of control electrodes;
   an AC power supply for applying a voltage between the pair of control electrodes; and
   a control impedance measuring device connected between the control electrode pairs.
C3. The sensor of claim C2, wherein the sensing impedance measuring device and the control impedance measuring device are disposed as an identical impedance measuring device.
D1. A sensor for detecting a measurement target substance contained in a solution, comprising:
   a sensing working electrode for detecting the measurement target substance;
   molecular chains arranged non-closely on a surface of the working electrode and configured to inhibit the non-measurement target substance;
   a counter electrode;
   a reference electrode; and
   an impedance measuring device connected to the sensing working electrode, the counter electrode and the reference electrode.
D2. The sensor of claim D1, further comprising a control working electrode connected to the impedance measuring device.

While several embodiments and examples of the present disclosure have been described above, these embodiments and examples illustrate the present disclosure. For example, each of the above-described embodiments has been described in detail in order to explain the present invention easily, and a circuit may be added as necessary. It is intended that the appended claims cover numerous modifications to the embodiments without departing from the spirit and scope of the present disclosure. Accordingly, the embodiments and examples disclosed herein have been shown by way of illustration and should not be considered as limiting the scope of the present disclosure.

### REFERENCE SIGNS LIST

100,200,300,400,500,600 Biosensor
101,201,701 Electrode
102,202,502,602,702 Molecular chain
103,203,303,403,503,603 solution
104,204,304,404,504,604 Container
105,205 Measurement target substance
106,206 Non-measuring target substance
107 Electrical circuit, electrical measuring circuit
207 Surface potential measuring device
211 Reference electrode
301,401 Working electrode
311,411 Reference electrode
321,421 Counter electrode
401 Working electrode
373,473,474 Current measuring circuit
371,471 Operational amplifier
372,472 Voltage power supply
401 Sensing electrode
431 Control working electrode
501 First electrode
502 First molecular chain
507 Impedance measuring device (measuring circuit)
511 Second electrode
512 Second molecular chain
601 First sensing electrode
611 Second sensing electrode
602 First molecular chain
612 Second molecular chain
671 Impedance measuring device for measurement (measuring circuit)
672 Impedance measuring device for control (measuring circuit)
621 First control electrode
631 Second control electrode
670 Electrical measuring circuit
703 Polymerization initiating molecular chain
704 Hydrophilic part

## Claims

1. A biosensor for detecting a measurement target substance contained in a solution, comprising:
a sensing electrode for electrically detecting the measurement target substance; and
molecular chains arranged non-closely on the surface of the sensing electrode and inhibiting a non-measurement target substance.

2. The sensor of claim 1, wherein the molecular chain comprises a hydrophilic polymer.

3. The sensor of claim 1 or 2, further comprising an electrical measurement circuit connected to the sensing electrode for detecting an electrical state of the electrode.

4. A sensor for detecting a measurement target substance contained in a solution, comprising:
a sensing electrode for electrically detecting the measurement target substance;
molecular chains arranged non-closely on a surface of the sensing electrode and configured to inhibit a non-measurement target substance;
a reference electrode; and
a surface potential measuring device connected to the electrode.

5. A sensor for detecting a measurement target substance contained in a solution, comprising:
a pair of sensing electrodes configured to bond with the measurement target substance;
molecular chains arranged non-closely on the sensing electrode and configured to inhibit a non-measurement target substance;
an AC power supply for applying a voltage between the pair of sensing electrodes; and
a sensing impedance measuring device connected between the pairs of sensing electrodes.

6. The sensor of claim 5, further comprising:
a pair of control electrodes;
an AC power supply for applying a voltage between the pair of control electrodes; and
a control impedance measuring device connected between the control electrode pairs.

7. The sensor of claim 6, wherein the sensing impedance measuring device and the control impedance measuring device are disposed as an identical impedance measuring device.

8. A sensor for detecting a measurement target substance contained in a solution, comprising:
a sensing working electrode for detecting the measurement target substance;
molecular chains arranged non-closely on a surface of the working electrode and configured to inhibit the non-measurement target substance;
a counter electrode;
a reference electrode; and
an impedance measuring device connected to the sensing working electrode, the counter electrode and the reference electrode.

9. The sensor of claim 8, further comprising a control working electrode connected to the impedance measuring device.

10. A biosensor for detecting a measurement target substance contained in a solution, comprising:
a sensing substrate; and
molecular chains arranged non-closely on a surface of the sensing substrate and configured to inhibit a non-measurement target substance from approaching the surface of the sensing substrate.
